(19) Europäisches Patentamt
European Patent Office
Office européen des brevets

(11) Veröffentlichungsnummer : **0 075 698**
**B1**

(12) # EUROPÄISCHE PATENTSCHRIFT

(45) Veröffentlichungstag der Patentschrift : **02.01.86**

(21) Anmeldenummer : **82107320.2**

(22) Anmeldetag : **12.08.82**

(51) Int. Cl.⁴ : **C 07 C 45/45**, C 07 C 49/675, C 07 C 49/697, C 07 C 49/755, C 07 C 69/757, C 07 C 67/313

(54) **Verfahren zur Herstellung von 10,11-Dihydro-5H-dibenzo (a,d) cyclohepten-5-on und dessen Substitutionsprodukten.**

(30) Priorität : **30.09.81 DE 3138842**
**31.10.81 DE 3143307**

(43) Veröffentlichungstag der Anmeldung : **06.04.83 Patentblatt 83/14**

(45) Bekanntmachung des Hinweises auf die Patenterteilung : **02.01.86 Patentblatt 86/01**

(84) Benannte Vertragsstaaten : **BE CH DE FR GB IT LI NL SE**

(56) Entgegenhaltungen :
**DE-C- 876 690**
**US-A- 2 879 297**
**US-A- 3 627 832**

(73) Patentinhaber : **DYNAMIT NOBEL AKTIENGESELLSCHAFT**
**Postfach 1209**
**D-5210 Troisdorf, Bez. Köln (DE)**

(72) Erfinder : **Zoche, Günter, Dr.**
**Königsheimstrasse 13**
**D-5300 Bonn 3 (DE)**
Erfinder : **Vogt, Wilhelm, Dr.**
**Kleiststrasse 39**
**D-5000 Köln 40 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 075 698 B1

**Beschreibung**

Verfahren zur Herstellung von 10,11-Dihydro-5H-dibenzo [a,d] cyclohepten-5-on und dessen Substitutionsprodukten.

Die Erfindung betrifft ein Verfahren zur Herstellung von 10,11-Dihydro-5H-dibenzo [a,d] 5-onen, besonders Dibenzosuberon (DBS), durch Cyclokondensation von Dibenzyl-o-carbonsäure bzw. deren Substitutionsprodukten.

DBS ist das Zwischenprodukt einer Reihe von Arzneimitteln, bei denen es sich im wesentlichen um Antidepressiva handelt. Die Substituenten der Arzneimittel bestimmen demgemäß Art und Menge der Substituenten im Ausgangsstoff und im Verfahrensprodukt.

Der kostengünstigste Syntheseweg zum DBS führt über Dibenzyl-o-carbonsäure (I).

Der Ringschluß der Dibenzyl-o-carbonsäure zum DBS kann entweder (A) über das Säurechlorid (II) mit anschließender Friedel-Crafts-Reaktion (J. Med. Pharm. Chem. *4*, 335-49 (1961) ; J. Am. Chem. Soc. *73*, 1668-73, (1951) ; Chem. Ber. *83*, (1950) 367-71) oder auf direktem Wege (B) mit Polyphosphorsäure (PPS) durchgeführt werden.

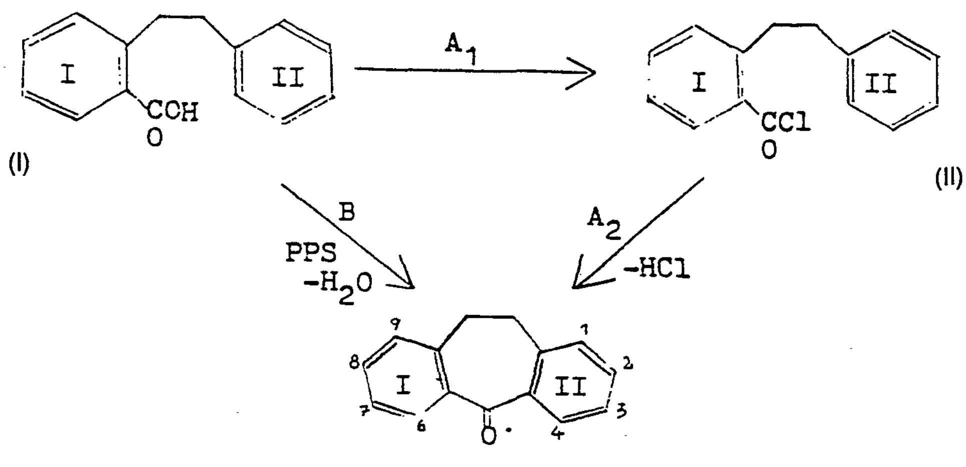


(I) (II)

DBS

Beim Weg A kann II nach den üblichen Methoden ($SOCl_2$, $PCl_3$ usw.) aus I hergestellt werden. In zweiter Stufe wird dann aus II mit Hilfe von meist großen Mengen an Friedel-Crafts-Katalysatoren DBS produziert.

Der Syntheseweg B ist zwar einstufig, hat aber den Nachteil, daß große Mengen von PPS, nämlich die 2 bis 50-fache Gewichtsmenge, benötigt werden, sodaß hohe Kosten für PPS entstehen. Da verdünnte Polyphosphorsäure nicht rentabel zu PPS aufgearbeitet werden kann, erfolgte eine erhebliche Belastung des Abwassers.

| | Literatur-Zitate | g PPS pro Mol Dibenzyl-o-carbonsäure | Reaktions- Temp. °C | Zeit h | DBS Ausbeute % |
|---|---|---|---|---|---|
| a | 1) 2) 3) 4) | 10 000 | 100 | 2 | 84 |
| b | 5) | X | 170 | 2,5 | 75 |
| c | 6) 7) | 1 565 | 170 | 3 | 91 |
| d | 8) | 350 | 120 | 2 | 97 |

X = Angaben fehlen.

1) US-PS 3.344.185
2) US-PS 3.459.859
3) US-PS 3.287.409
4) US-PS 3.369.044
5) US-PS 3.052.721
6) Galenica Acta *15* (2), 77-87 (1962)
7) Helv. Chim. Acta *36*, 1489-99 (1953)
8) Synthesis (1972) 612-4

Nachteilig ist bei Weg B wie auch Weg A besonders die Notwendigkeit der Behandlung mit Wasser,

um den Friedel-Crafts-Katalysator bzw. die PPS zu entfernen und die DBS isolieren zu können sowie die Erfordernis, ein oder mehrere organische Lösungsmittel zu verwenden.

Es bestand daher die Aufgabe, Dibenzyl-o-carbonsäure und dessen Substitutionsprodukte einfach und rentabel zu DBS und dessen Substitutionsprodukte zu cyclisieren.

Gegenstand der Erfindung ist ein Verfahren zur Herstellung von 10,11-Dihydro-5H-dibenzo [a,d] cyclohepten-5-on und dessen Substitutionsprodukte

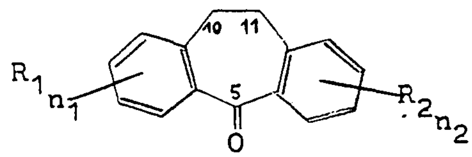

worin Wasserstoff teilweise durch $R_1$ und/oder $R_2$ substituiert sein kann und $R_1$ und $R_2$ für

Halogen und/oder
Alkyl- und/oder
Alkoxy- und/oder
Aryl- und/oder
Aralkyl- und/oder

Carboalkoxygruppen steht und $n_1$ bzw. $n_2$ die Zahlenwerte 0, 1, 2, 3 oder 4 hat, durch Cyclokondensation der entsprechenden Dibenzyl-o-carbonsäuren, dadurch gekennzeichnet, daß man Dibenzyl-o-carbonsäuren mit Aromaten, die eine oder mehrere kerngebundene Di- und/oder Trichlormethylgruppen enthalten, in Gegenwart einer Lewissäure als Katalysator bei Temperaturen zwischen 70 und 150 °C umsetzt.

Die Umsetzung erfolgt gemäß folgender Gleichung, bei der als Trichlormethylaromat Benzotrichlorid gewählt wurde :

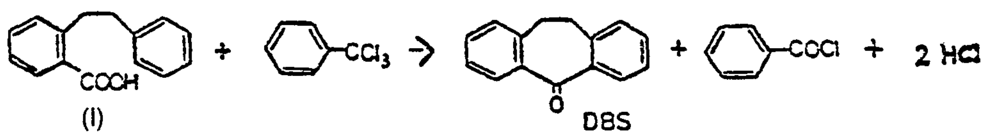

(I) DBS

Als Katalysatoren lassen sich eine große Anzahl von Lewissäuren einsetzen. Besonders geeignet sind die als Lewissäuren wirkenden Eisen- und Zinkverbindungen wie z. B. wasserfreies Eisen (III) Chlorid, Zinkchlorid oder Eisen (III) Phosphat. Die Katalysatormenge ist in weiten Grenzen variierbar. Bereits Mengen von 0,01 Gew.-%, bezogen auf die Dibenzylcarbonsäure, zeigen eine katalytische Wirkung. Da jedoch eine gewisse Abhängigkeit zwischen Katalysatormenge und Reaktionsgeschwindigkeit besteht, wählt man aus ökonomischen Gründen 0,02 bis 5 Gew.-% Katalysator, bezogen auf die Dibenzylcarbonsäure. Es sind jedoch auch größere Mengen, z. B. bis zu 10 Gew.-% erfindungsgemäß einsetzbar.

Die Reaktion findet bei erhöhter Temperatur statt. Der Beginn der Reaktion läßt sich an der auftretenden HCl-Entwicklung feststellen. In diesem Temperaturbereich, der im allgemeinen bei ca. 70 °C beginnt, läßt man zweckmäßigerweise zuerst die Reaktion ablaufen und erhöht dann bei abnehmender Gasentwicklung allmählich die Temperatur auf 120 bis 150 °C. In diesem Temperaturbereich wird die Reaktion zu Ende geführt. Prinzipiell ist es jedoch auch möglich, die gesamte Umsetzung bei 120 bis 150 °C durchzuführen. Nach Beendigung der HCl-Abspaltung läßt sich das Dibenzosuberon von dem als Nebenprodukt anfallenden Säurechlorid durch fraktionierte Destillation direkt erhalten. Eine weitere Aufarbeitung ist nicht nötig.

Es ist bekannt, daß Trichlormethylaromaten mit Carbonsäuren unter Bildung der Säurechloride der Carbonsäure reagieren. Überraschend ist jedoch, daß im vorliegenden Fall die zu erwartenden Säurechloride der Dibenzyl-o-carbonsäuren unter HCl-Abspaltung sofort zum entsprechenden Dibenzosuberon cyclisieren. Überraschend ist auch, daß diese Reaktion schon durch die genannten geringen Mengen von z. B. Zink- oder Eisenverbindungen katalysiert wird.

Die erfindungsgemäß einsetzbaren Chlormethylaromaten können ein- oder mehrkernig sein und eine oder mehrere Di- oder Trichlormethylgruppen besitzen. Bevorzugt werden einkernige Verbindungen mit einer oder mehreren Trichlormethylgruppen eingesetzt. Als Beispiel seien Benzotrichlorid, 1,4-Bis-(trichlormethyl)benzol und 1-Trichlormethyl-2-dichlormethylbenzol erwähnt. Bei der zuletzt genannten Verbindung reagiert auch die Dichlormethylgruppe entsprechend der oben genannten Reaktion. In diesem Falle resultiert 3-Chlorphthalid.

Die Chlormethylaromaten werden bevorzugt im stöchiometrischen Verhältnis zur Dibenzylcarbonsäure eingesetzt. Die Stöchiometrie bezieht sich dabei auf die Anzahl der reaktionsfähigen Di- oder Trichlormethylgruppen des Chlormethylaromaten. Diese setzen sich bei der Reaktion zu Endprodukten um, die ebenfalls in großer Reinheit ohne zusätzliche Reinigungsoperationen bei der fraktionierten Vakuumdestillation erhalten werden. Die dabei gewonnenen Produkte, z. B. Benzoylchlorid, Terephthalsäuredichlorid oder 3-Chlorphthalid sind ebenfalls wichtige Zwischenprodukte in der chemischen und

pharmazeutischen Industrie. Das erfindungsgemäße Verfahren hat demzufolge gegenüber den bekannten Verfahren die zusätzlichen Vorteile, daß Nebenprodukte entstehen, die erstens verwertbar sind und zweitens nicht erst in zusätzlichen Verfahrensschritten aufgearbeitet werden müßten.

Ein weiterer Vorteil des Verfahrens ist die hohe Reinheit des anfallenden Chlorwasserstoffs, da tiefsiedende Verbindungen, wie z. B. $SO_2$ oder $SOCl_2$, nicht zugegen sind; dadurch wird eine Weiterverwendung des anfallenden Chlorwasserstoffs als Synthesegas erheblich erleichtert.

Weder die Reaktion noch die Reinigung bzw. Isolierung der Reaktionsprodukte wird im allgemeinen unter Verwendung von Lösungsmitteln durchgeführt. Somit enffallen auch alle bei den bekannten Verfahren notwendigen Extraktionsschritte zur Abtrennung des Lösungsmittels, das anschließend entweder vernichtet oder wieder aufgearbeitet werden müßte.

Falls es jedoch unter speziellen Bedingungen notwendig ist, in einem Lösungsmittel zu arbeiten, so ist dies erfindungsgemäß ebenfalls möglich. Als Lösungsmittel eignen sich dann gegenüber den Reaktionsbedingungen inerte Lösungsmittel, deren Siedetemperaturen über 100 °C, vorzugsweise über 150 °C, liegen, wie z. B. Nitrobenzol.

Die Ausgangsstoffe können bis zu 8, in jedem der Ringe I und II bis zu 4 Substituenten, tragen. Die Substituenten sollen bevorzugt bei der Reaktion inert sein, jedoch ist auch eine chemische Veränderung der Abspaltung der Substituenten möglich, sofern die Cyclisierung nicht behindert ist.

Bevorzugtes Produkt ist Dibenzosuberon und in zweiter Linie Substitutionsprodukte mit einem oder zwei Substituenten. Bevorzugte Substituenten sind Methoxy, Alkyl von 1 bis 4 Kohlenstoffatomen und Chlor. m-Substituenten im Ring II der Ausgangsstoffe treten im Produkt in 2-Stellung auf.

## Beispiel 1

In einem 100 ml-Glaskolben werden unter Feuchtigkeitsausschluß und Rühren 22,6 g Dibenzyl-o-carbonsäure, 19,5 g Benzotrichlorid und 1,0 g $FeCl_3$ (wasserfrei) zunächst auf 80 °C erhitzt. Bei Nachlassen der bei dieser Temperatur auftretenden HCl-Entwicklung wird die Temperatur allmählich auf 125 °C erhöht. Die Umsetzung ist nach insgesamt 2 h beendet. Anschließend wird der Kolbeninhalt im Vakuum destilliert. Die Fraktion $Kp_{0,2}$: 136-40 °C besteht aus 18,0 g Dibenzosuberon mit einer GC-Reinheit von 96,6 %.

## Beispiel 2

Apparatur wie in Beispiel 1.

Es werden 22,6 g Dibenzyl-o-carbonsäure, 19,5 g Benzotrichlorid und 1,0 g $ZnCl_2$ (wasserfrei) zunächst auf 80 °C erhitzt. Bei Nachlassen der HCl-Entwicklung wird die Temperatur allmählich auf 150 °C erhöht. Die Umsetzung ist nach insgesamt 4,5 h beendet. Anschließend wird der Kolbeninhalt im Vakuum destilliert. Die Fraktion $Kp_{0,2}$: 136-40 °C besteht aus 15,3 g Dibenzosuberon mit einer GC-Reinheit von 95,1 %.

## Beispiel 3

Apparatur wie in Beispiel 1.

Es werden 22,6 g Dibenzyl-o-carbonsäure, 19,5 g Benzotrichlorid und 50 mg $FePO_4$ (wasserfrei) zunächst auf 100 °C erhitzt. Bei Nachlassen der HCl-Entwicklung wird die Temperatur allmählich auf 150 °C erhöht. Die Umsetzung ist nach insgesamt 3 h beendet. Die Fraktion $Kp_{0,2}$: 136-40 °C besteht aus 17,0 g Dibenzosuberon mit einer GC-Reinheit von 97,3 %.

## Beispiel 4

Apparatur wie in Beispiel 1.

Es werden 22,6 g Dibenzyl-o-carbonsäure, 13,9 g l-Trichlormethyl-2-dichlormethylbenzol und 1,0 g $FeCl_3$ (wasserfrei) zunächst auf 100 °C erhitzt. Bei Nachlassen der HCl-Entwicklung wird die Temperatur allmählich auf 120 °C erhöht. Die Umsetzung ist nach insgesamt 6,25 h beendet. Anschließend wird der Kolbeninhalt im Vakuum destilliert. Die Fraktion $Kp_{0,2}$: 136-40 °C besteht aus 12,4 g Dibenzosuberon mit einer GC-Reinheit von 95,8 %.

## Beispiel 5

Apparatur wie in Beispiel 1.

Es werden 22,6 g Dibenzyl-o-carbonsäure, 19,5 g Benzotrichlorid, 1,0 $FeCl_3$ (wasserfrei) und 70 ml Nitrobenzol zunächst auf 80 °C erhitzt. Bei Nachlassen der HCl-Entwicklung wird die Temperatur allmählich auf 110 °C erhöht. Die Umsetzung ist nach insgesamt 2 h beendet. Anschliessend wird der Kolbeninhalt im Vakuum destilliert. Die Fraktion $Kp_{0,2}$: 136-40 °C besteht aus 20 g Dibenzosuberon mit einer GC-Reinheit von 92,2 %.

Beispiel 6

Beispiel 1 wird mit der äquivalenten Menge 2-[2-(m-Methoxyphenyl)-ethyl]-benzoesäure (Fp. 117 bis 119 °C) anstelle von Dibenzyl-o-carbonsäure, wiederholt, wobei in entsprechender Ausbeute 2-Methoxy-10,11-dihydro-5H-dibenzo [a,d] cyclohepten-5-on (Fp. 71 bis 73 °C aus Wasser/Ethanol) entsteht.

Beispiele 7 und 8

Beispiel 1 wird mit äquivalenten Mengen von a) 2-[2-p-Methylphenyl)-ethyl]-benzoesäure (Fp. 82 °C) und b) 2-[2-p-Chlorphenyl)-ethyl]-benzoesäure (Fp. 122 °C) wiederholt, wobei gute Ausbeuten von a) 3-Methyl-10,11-dihydro-5H-dibenzo [a,d] cyclohepten-5-on (Fp. 34 bis 35 °C) bzw. b) 3-Chlor-10,11-dihydro-5H-dibenzo [a,d] cyclohepten-5-on (Fp. 64 bis 65 °C) entstehen.

**Patentansprüche**

1. Verfahren zur Herstellung von 10,11-Dihydro-5H-dibenzo [a,d] cyclohepten-5-on und dessen Substitutionsprodukten der Formel

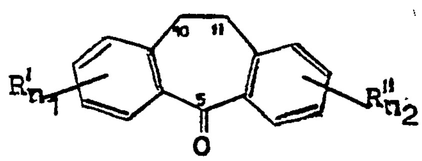

worin Wasserstoff teilweise durch R′ und/oder R″ substituiert sein kann, und R′ und R″ für
Halogen und/oder
Alkyl- und/oder
Alkoxy- und/oder
Aryl- und/oder
Aralkyl- und/oder
Carboalkoxygruppen steht und $n_1$ bzw. $n_2$ die Zahlenwerte 0, 1, 2, 3 oder 4 hat, durch Cyclokondensation der entsprechenden Dibenzyl-o-carbonsäuren, dadurch gekennzeichnet, daß man Dibenzyl-o-carbonsäuren mit Aromaten, die eine oder mehrere kerngebundene Di- und/oder Trichlormethylgruppen enthalten, in Gegenwart einer Lewissäure als Katalysator bei Temperaturen zwischen 70 und 150 °C umsetzt.

2. Verfahren nach Anspruch 1, dadurch gekennzeichnet, daß man als Lewissäuren Verbindungen von Zink und/oder Eisen einsetzt.

3. Verfahren nach Anspruch 1 oder 2, dadurch gekennzeichnet, daß man den Katalysator in Mengen von 0,01 bis 10 Gew.-%, vorzugsweise zwischen 0,02 und 5 %, bezogen auf die Dibenzylcarbonsäure, einsetzt.

4. Verfahren gemäß einem der Ansprüche 1 bis 3, dadurch gekennzeichnet, daß als Aromaten, die kerngebundene Di- oder Trichlormethylgruppen enthalten, einkernige Aromaten eingesetzt werden.

5. Verfahren gemäß einem der Ansprüche 1 bis 4, dadurch gekennzeichnet, daß die Aromaten, die kerngebundene Trichlormethylgruppen enthalten, in stöchiometrischer Menge, bezogen auf die Dibenzylcarbonsäure, eingesetzt werden.

6. Verfahren gemäß einem der Ansprüche 1 bis 5, dadurch gekennzeichnet, daß die Umsetzung vorzugsweise bei Temperaturen zwischen 75 und 150 °C durchgeführt wird.

**Claims**

1. Process for the preparation of 10,11-dihydro-5H-dibenzo [a,d] cyclohepten-5-one and its substitution product of the formula

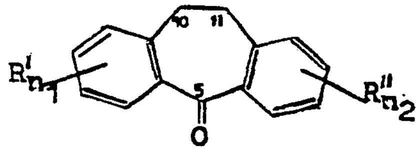

wherein hydrogen can be substituted partially by R′ and/or R″, and R′ and R″ stand for
halogen and/or
alkyl and/or
alkoxy- and/or

aryl- and/or
aralkyl- and/or
carboalkoxy groups and $n_1$ or $n_1$ has the numerical values 0, 1, 2, 3 or 4, by cyclocondensation of the corresponding dibenzyl-o-carboxylic acids, characterised in that dibenzyl-o-carboxylic acids are reacted with aromatic compounds which contain one or several nuclearly bound di- and/or trichloromethyl groups, in the presence of a Lewis acid as catalyst at temperatures between 70 and 150 °C.

2. Process according to claim 1, characterised in that compounds of zinc and/or iron are employed as Lewis acids.

3. Process according to claim 1 or 2, characterised in that the catalyst is employed in amounts of 0.01 to 10 % by weight, preferably between 0.02 and 5 %, related to the dibenzyl carboxylic acid.

4. Process according to one of claims 1 to 3, characterised in that mononuclear aromatic compounds are employed as aromatic compounds which contain nuclearly bound di- or trichloromethyl groups.

5. Process according to one of claims 1 to 4, characterised in that the aromatic compounds which contain nuclearly bound trichloromethyl groups are employed in stoichiometric amounts related to the dibenzyl carboxylic acid.

6. Process according to one of claims 1 to 5, characterised in that the reaction is preferably carried out at temperatures between 75 and 150 °C.

## Revendications

1. Procédé pour la préparation de 10,11-dihydro-5H-dibenzo [a,d] cycloheptène-5-one et de ses produits de substitution de formule

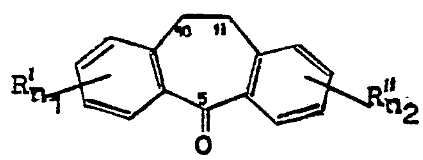

dans laquelle l'hydrogène peut être partiellement substitué par R' et/ou R'', R' et R'' sont un halogène
et/ou des groupes alkyles,
et/ou des groupes alcoxy,
et/ou des groupes aryles,
et/ou des groupes aralkyles,
et/ou des groupes carboalcoxy
et $n_1$, respectivement $n_2$ possède des valeurs numériques 0, 1, 2, 3 ou 4, par cyclocondensation des acides dibenzyl-o-carboxyliques correspondants, caractérisé en ce qu'on fait réagir des acides dibenzyl-o-carboxyliques avec des hydrocarbures aromatiques renfermant un ou plusieurs groupes di- et/ou trichlorométhyle fixés sur le noyau, en présence d'un acide de Lewis comme catalyseur à des températures entre 70 et 150 °C.

2. Procédé selon la revendication 1, caractérisé en ce qu'on utilise des composés de zinc et/ou de fer comme acides de Lewis.

3. Procédé selon la revendication 1 ou 2, caractérisé en ce qu'on emploie le catalyseur en quantités de 0,01 à 10 % en poids, de préférence à raison de 0,02 à 5 %, par rapport à l'acide dibenzylcarboxylique.

4. Procédé selon l'une quelconque des revendications 1 à 3, caractérisé en ce qu'on utilise comme hydrocarbures aromatiques renfermant des groupes di- ou trichlorométhyle fixés sur le noyau des hydrocarbures aromatiques monocycliques.

5. Procédé selon l'une quelconque des revendications 1 à 4, caractérisé en ce que les hydrocarbures aromatiques renfermant des groupes trichlorométhyle fixés au noyau sont utilisés en une quantité stœchiométrique par rapport à l'acide dibenzylcarboxylique.

6. Procédé selon l'une quelconque des revendications 1 à 5, caractérisé en ce que la réaction est conduite de préférence à des températures entre 75 et 150 °C.